(19)

Europäisches Patentamt
European Patent Office
Office européen des brevets

(11) **EP 4 417 128 A1**

(12) **EUROPEAN PATENT APPLICATION**
published in accordance with Art. 153(4) EPC

(43) Date of publication:
**21.08.2024 Bulletin 2024/34**

(21) Application number: **22880766.5**

(22) Date of filing: **27.09.2022**

(51) International Patent Classification (IPC):
*A61B 5/37* (2021.01)     *A61B 5/271* (2021.01)
*A61B 5/293* (2021.01)     *A61N 1/05* (2006.01)
*A61N 1/36* (2006.01)

(52) Cooperative Patent Classification (CPC):
**A61B 5/293; A61B 5/263; A61B 5/37; A61N 1/05; A61N 1/36;** A61B 2562/125; A61B 2562/164; A61B 2562/166

(86) International application number:
**PCT/JP2022/035817**

(87) International publication number:
**WO 2023/063070 (20.04.2023 Gazette 2023/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.10.2021  JP 2021167887**

(71) Applicant: **Tokyo Institute of Technology Tokyo 152-8550 (JP)**

(72) Inventors:
• **FUJIE, Toshinori**
**Tokyo 152-8550 (JP)**
• **MIYASHITA, Eizo**
**Tokyo 152-8550 (JP)**
• **IMAI, Ayano**
**Tokyo 152-8550 (JP)**

(74) Representative: **Angerhausen, Christoph Boehmert & Boehmert Anwaltspartnerschaft mbB Pettenkoferstrasse 22 80336 München (DE)**

(54) **NEURAL ELECTRODE FOR RECORDING AND/OR STIMULATION PURPOSES WHICH IS INTENDED TO BE ADHERED TO BRAIN SURFACE UPON USE, AND RECORDING OR STIMULATION METHOD USING SAME**

(57)     Provided are a recording and/or stimulation neural electrode that is flexible and can be tightly attached to the brain surface, and shows no deterioration in adhesiveness even when embedded for a long period of time; and a method using such neural electrode. A neural electrode 1 of the present invention is such that its attaching part 2 which is a sheet-shaped electrode is to be attached to the brain surface upon use, wherein the attaching part has at least:
an insulating sheet 3 whose one surface serves as an attaching surface to be attached to the brain surface; and a conductive wiring 4 formed on a surface of the insulating sheet that is opposite to the attaching surface, wherein

multi-point electrode parts 4a of the conductive wiring are exposed from hole parts 3a of the insulating sheet, and wherein
the insulating sheet is an elastomer thin film having a thickness of 2 to 100 $\mu$m, and the conductive wiring has a thickness of 10 $\mu$m or smaller.

FIG.4A

Production of SBS sheet (4$\mu$m, bar coating)

EP 4 417 128 A1

**Description**

TECHNICAL FIELD

The present invention relates to a recording and/or stimulation neural electrode to be attached to the brain surface upon use; and a recording or stimulation method using such electrode.

BACKGROUND ART

Electrocorticography (ECoG) electrodes capable of measuring nerve potentials when attached to the brain surface are used to identify lesions of intractable epilepsy for which antiepileptic drugs are ineffective. However, current ECoG electrodes have problems with misalignment and cerebral hypertension due to mechanical mismatch between electrode base materials and brain tissues.

Further, there has been developed a system for detecting and suppressing epileptic seizures by immediately stimulating the epileptic lesions with electricity (Responsive Neuro Stimulation (RNS)). This system is applied in clinical settings by NeuroPace, Inc. in the U.S. However, a bioadhesive property will be impaired if embedded for a long period of time. For patients with intractable epilepsy for which antiepileptic drugs are ineffective, there is a demand for a technique to place multiple cortical electrodes over a large area of the brain surface without compressing the brain and without having these electrodes misaligned in order to identify epileptic lesions and treat seizures via local electrical stimulation for a long period of time.

These problems are caused by the large film thicknesses of the electrodes. As conventional products, although there are also neural electrodes using silicone rubbers as base materials, they have similar problems in terms of both base material and wiring. As techniques related to electrodes to be attached to the brain surface, there are known, for example, a technique using a soluble silk fibroin film as a base material for forming an electrode (Non-patent document 1); and a technique using a paraxylylene-based polymer (Non-patent document 2). However, adhesion to the brain surface remains an issue in this technical field. Recently, there has been developed an electrode using hydrogel as a base material (Non-patent document 3).

This electrode has a conformability to the brain surface in the acute stage. However, if embedded for a long period of time, its bioadhesiveness will be impaired due to deformation of the material (e.g., swelling of the gel).

Meanwhile, there has been proposed a technique using a flexible polymer nanosheet as a method of directly fixing an electronic device to arms, legs and the like (Patent document 1). However, there is no disclosure regarding its applicability to a recording and/or stimulation neural electrode that is to be attached to the brain surface, or a suitable configuration for this purpose.

PRIOR ART DOCUMENTS

Non-patent documents

Non-patent document 1: NATURE MATERIALS j VOL 9 j JUNE 2010 j www.nature.com/naturematerials
Non-patent document 2: Scientific Reports | (2018) 8:3825| DOI: 10.1038/s41598-018-22051-z
Non-patent document 3: Scientific Reports | (2019) 9:13379| https://doi.org/10.1038/s41598-019-49772-z

Patent documents

Patent document 1: Pamphlet of WO2016/181958

SUMMARY OF THE INVENTION

Problems to be solved by the invention

As one use case, in the diagnosis and treatment of intractable epilepsy, the current medical examination flow involves implanting intracranial electrodes, and performing recording with a reduced dosage and via a wired connection for approximately two weeks. During this period, electroencephalography analysis is conducted by a manual operation and visual observation. For this reason, a device implantation surgery is required when performing a surgical operation or neuromodulation. As a burden on patients, they must be hospitalized and managed with intracranial electrodes being implanted and wired to an electroencephalograph. Further, there is a risk of generalized convulsion and status epilepticus when patients undergo chronic intracranial electroencephalography while on reduced doses of antiepileptic drugs. As the burden on medical personnel, there is a need to manually and visually analyze the vast amount of brain wave data obtained within a limited period of time to formulate a treatment plan. In addition, chronic intracranial electroencephalography is performed with patients being on reduced doses of antiepileptic drugs, which requires dedicated observation staff to ensure the patients' safety.

In contrast to such long-term hospitalization management, the future medical examination flow aims to realize an automatic electroencephalography analysis via a wireless establishment enabled by full implantation. In such future medical examination flow, after implanting an intracranial electrode-containing device, and then after hospitalization for a short period of time, recordings can then be performed for one up to six months at home under normal conditions without reduction in dosages. Such medical examination flow is expected to reduce the burden on patients and medical personnel. If an integrated device including an implantable device and a remote monitoring system is realized, the device will have a clinical significance in that it can be used continuously for treatment purposes from the moment of diagnosis without replacing electrodes. Moreover, since there is no need to perform re-operation as the device is implanted at the time of a surgical operation or neuromodulation, there are expected two benefits of improving treatment outcomes for intractable epilepsy and reducing the burden on patients and medical personnel.

However, conventional electrodes have a problem of being thick and hard, making it difficult to stably place a plurality of such electrodes, and have them embedded for a long period of time as brain tissues will be compressed thereby. These difficulties have imposed restrictions on the number of electrodes implanted and an area of implantation. This is a factor that makes application to the abovementioned future medical examination flow difficult.

The present invention was made in view of the aforementioned problems. It is an object of the present invention to provide a recording and/or stimulation neural electrode that is flexible and can be tightly attached to the brain surface, and shows no deterioration in adhesiveness even when embedded for a long period of time; and a method using such neural electrode.

Means to solve the problems

The inventors of the present invention diligently conducted a series of studies to solve the above problems, and completed the invention as follows. Specifically, the inventors produced a thin film electrode combining an elastomer polymer thin film and printed electronics. As a result, they found that this thin film electrode was flexible and could be tightly attached to the brain surface, and was capable of recording nerve potentials or the like and performing electrical stimulation. That is, the neural electrode of the present invention is a recording and stimulation electrode formed thinner, lighter, and softer than the conventional electrodes. These features allow a plurality of such electrodes to be stably placed and embedded for a long period of time without compressing brain tissues. For this reason, brain wave recording in a wide range and at a high density is made possible. The electrode of the invention is also suitable for embodying a wireless remote monitoring system, which is a goal pursued by the medical examination flow for diagnosis and treatment of intractable epilepsy, etc.

Particularly, the following invention is disclosed.

[1] A recording and/or stimulation neural electrode with an attaching part thereof as a sheet-shaped electrode being attached to the brain surface upon use, wherein

the attaching part has at least:

an insulating sheet whose one surface serves as an attaching surface to be attached to the brain surface; and
a conductive wiring formed on a surface of the insulating sheet that is opposite to the attaching surface, wherein

multi-point electrode parts of the conductive wiring are exposed from hole parts of the insulating sheet, and wherein
the insulating sheet is an elastomer thin film having a thickness of 2 to 100 $\mu$m, and the conductive wiring has a thickness of 10 $\mu$m or smaller.

[2] The recording and/or stimulation neural electrode according to [1], wherein the neural electrode has a base material sheet on which the conductive wiring is formed, the base material sheet is tightly attached to the insulating sheet across the conductive wiring at the attaching part, and the base material sheet is an elastomer thin film having a thickness of 100 $\mu$m or smaller.

[3] The recording and/or stimulation neural electrode according to [2], wherein the conductive wiring is a printed wiring.

[4] The recording and/or stimulation neural electrode according to [3], wherein the base material sheet is hydrophilized on the conductive wiring side.

[5] The recording and/or stimulation neural electrode according to any one of [1] to [4], wherein the insulating sheet is a styrene-based elastomer.

[6] The recording and/or stimulation neural electrode according to any one of [1] to [5], wherein the attaching part has a bending stiffness of $1 \times 10^{-3}$ Nm or lower.

[7] The recording and/or stimulation neural electrode according to any one of [1] to [6], wherein a support film supporting the base material sheet is tightly attached to an area that is continuously extended from the attaching part toward the opposite side of the multi-point electrode parts.

[8] A method of recording weak electric potentials or electric currents associated with brain activity, or stimulating the brain by transmitting electric currents to the brain, comprising:

a step of attaching the neural electrode according to any one of [1] to [7] to the brain surface so that the multi-point electrode parts are in contact with the brain surface; and

a step of receiving weak electric potentials or electric currents associated with brain activity by the multi-point electrode parts and recording the electric potentials or electric currents by a measurement device electrically connected to the conductive wiring, or stimulating the brain by supplying and transmitting electric currents from a stimulator electrically connected to the conductive wiring to the brain.

Effects of the invention

According to the recording and/or stimulation neural electrode of the present invention and the method using the same, the neural electrode is flexible and can be tightly attached to the brain surface, and shows no deterioration in adhesiveness even when embedded for a long period of time.

BRIEF DESCRIPTION OF THE DRAWINGS

FIG. 1 is a top view (back view) schematically showing one embodiment of a neural electrode of the present invention.

FIG.2 is a bottom view of the neural electrode in FIG. 1 (surface to be attached to the brain surface).

FIG.3 is a set of cross-sectional views where FIG.3A is an enlarged partial cross-sectional view taken on a line A-A in FIG. 1, and FIG.3B is an enlarged partial cross-sectional view taken on a line B-B in FIG. 1.

FIG.4 is a diagram schematically showing an example of a procedure for producing the neural electrode of the present invention.

FIG.5 is a set of photographs showing how printed wirings are formed on SBS thin films that have been subjected to plasma treatment for different lengths of time in a working example.

FIG.6 is a diagram schematically showing a measurement system for an insulating layer in a working example.

FIG.7 is a graph showing resistivities with SBS thin films having various film thicknesses measured by the measurement system in FIG.6.

FIG.8 is a diagram schematically showing a resistivity measurement system in a working example.

FIG.9 is a graph showing rates of change in conducting wire resistance to reciprocals of conducting wire widths, that is measured by the measurement system in FIG.8.

FIG. 10 is a graph showing a measurement result of the impedance of an electrode in a working example (electrode diameter 250 $\mu$m).

FIG. 11 is a series of drawings illustrating a stimulation potential measurement experiment utilizing a whisker-brain response circuit in a working example, where FIG. 11A is a photograph showing a state in which the electrode is attached to the brain surface of a rat, and FIGs.11B and 11C are diagrams schematically showing a correlation between whisker stimulation sites and a multi-point electrode array ($4 \times 4$).

FIG.12 is a series of graphs showing results of a stimulation potential measurement carried out using the measurement system illustrated in FIG. 11.

FIG.13 is a diagram schematically showing a current load test system in a working example.

FIG.14 is a graph showing an output current waveform measured using the measurement system in FIG. 13.

FIG.15 is a graph showing the muscle potentials of whiskers being electrically stimulated, that are measured by attaching the electrode of the working example to the brain surface of a rat, where a stimulus current is indicated by a bold straight line; and as for EMG, recordings of 46 trials are respectively indicated by thin curved lines, and an average thereof is indicated by a bold curved line.

FIG.16 is a photograph showing a state where the electrode of the working example is attached to a brain gel model.

MODE FOR CARRYING OUT THE INVENTION

The following is a description of a specific embodiment of the present invention with reference to the accompanying drawings. Here, the drawings are provided for the purpose of illustrating the embodiment, and are not intended to show the actual dimensions thereof; please refer to the descriptions in the specification.

In the present invention, the thicknesses of composing elements of a neural electrode, such as an insulating sheet, a substrate sheet, and a conductive wiring, are average values determined using a surface profiler or other measurement device.

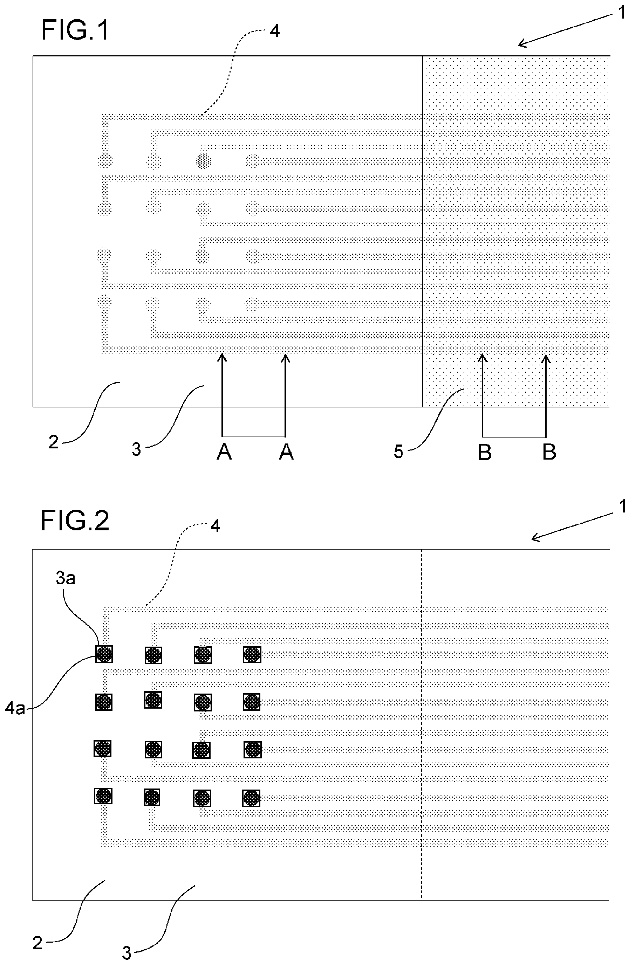

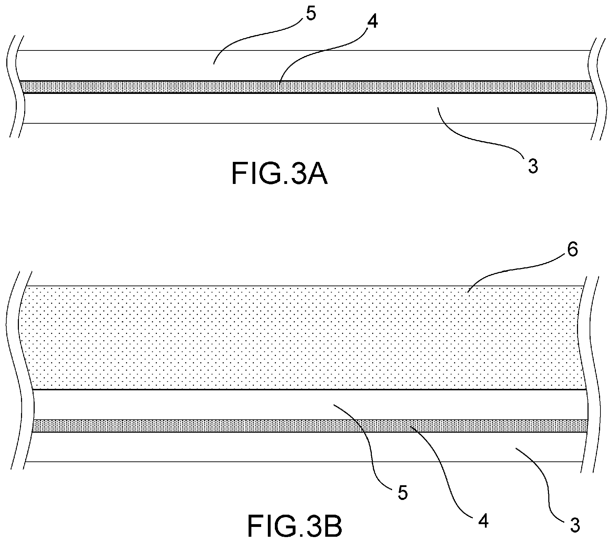

FIG. 1 to FIG.3 are drawings schematically showing one embodiment of the neural electrode of the present invention. Specifically, FIG. 1 is a top view (back view), FIG.2 is a bottom view (surface to be attached to the brain surface), FIG.3(A) is an enlarged partial cross-sectional view taken on a line A-Ain FIG. 1, and FIG.3(B) is an enlarged partial cross-sectional view taken on a line B-B in FIG. 1.

A neural electrode 1 of this embodiment is a recording and/or stimulation neural electrode used by attaching an attaching part 2 as a sheet-shaped electrode to the brain surface. The attaching part 2 has at least an insulating sheet 3 whose one surface serves as an attaching surface to be attached to the brain surface, and a conductive wiring 4 formed on a surface of the insulating sheet 3 that is opposite to the attaching surface 2. Multi-point electrode parts 4a of the conductive wiring 4 are exposed from hole parts 3a of the insulating sheet 3. The insulating sheet 3 is an elastomer thin film having a thickness of 2 to 100 $\mu$m, and the conductive wiring 4 has a thickness of 10 $\mu$m or smaller.

The neural electrode 1 has a base material sheet 5 on which the conductive wiring 4 is formed. The base material sheet 5 is tightly attached to the insulating sheet 3 across the conductive wiring 4 at the attaching part 2, and the base material sheet 5 is an elastomer thin film having a thickness of 100 $\mu$m or smaller.

The neural electrode 1 is for recording and/or stimulation. Specifically, weak electric potentials associated with brain activity are received by the electrode parts 4a at multiple points, and are recorded by a measurement device electrically connected to the conductive wiring 4. Examples of applications include intracranial electrodes for epileptic focal diagnosis and electrodes for brain-machine interface (BMI). In particular, the neural electrode 1 can be used as a recording neural electrode with a high spatial distribution density as required for BMI. In addition, an electric current is supplied and transmitted to the brain from a stimulator electrically connected to the conductive wiring 4 so as to stimulate the brain. For example, the neural electrode 1 may be applied to a system that suppresses epileptic seizures by immediately and electrically stimulating epileptic lesion upon detecting epileptic seizures, and to neuromodulation.

As an intracranial electrode, the neural electrode 1 can be used, for example, as a subdural electrode that is implanted in the subdural brain surface. The subdural electrode is an electrode used to detect brain waves at the brain surface, and can also be used as an electrode for brain stimulation.

The insulating sheet 3 is an elastomer thin film having a thickness of 2 to 100 $\mu$m. When the thickness of the insulating sheet 3 is 100 $\mu$m or smaller, it is flexible and can be tightly attached to the brain surface. When the thickness of the insulating sheet 3 is 2 $\mu$m or larger, the conductive wiring 4 can be securely insulated from the brain surface. From these perspectives, the upper limit of the thickness of the insulating sheet 3 is preferably 90 $\mu$m or smaller, more preferably 70 $\mu$m or smaller, even more preferably 50 $\mu$m or smaller, and particularly preferably 30 $\mu$m or smaller. The lower limit of the thickness of the insulating sheet 3 is preferably 3 $\mu$m or larger, preferably 4 $\mu$m or larger.

An elastomer as the material of the insulating sheet 3 is not particularly limited as long as it is safe and compatible for use when in contact with and attached to the brain surface. There may be used an elastic polymer such as a thermoplastic or a thermosetting elastomer.

Specifically, there may be listed, for example, a styrene-based elastomer, a silicone-based elastomer, an olefin-based elastomer, an ester-based elastomer, a urethane-based elastomer, an amide-based elastomer, and a vinyl chloride-based elastomer. Any one kind of them may be used alone, or two or more kinds of them may be used in combination. Also, a polymer other than an elastomer, such as a polylactic acid, may be used in combination as the material for the insulating sheet 3.

Examples of a styrene-based elastomer include a styrene-butadiene-styrene block copolymer (SBS), a styrene-ethylene-butylene-styrene block copolymer (SEBS), a styrene-isoprene-styrene block copolymer (SIS), a styrene-ethylene-propylene-styrene block copolymer (SEPS, hydrogenated product of SIS), a styrene-ethylene-propylene block copolymer (SEP, hydrogenated product of styrene-isoprene block copolymer), and a styrene-isobutylene-styrene block copolymer (SIBS).

A silicone-based elastomer is mainly composed of organopolysiloxane, examples of which may include polydimethylsiloxane-based, polymethylphenylsiloxane-based, and polydiphenylsiloxane-based elastomers. They may be partially modified with vinyl groups, alkoxy groups or the like. A thin film of an organopolysiloxane is obtained by, for example, treating a main agent containing a siloxane compound with a curing agent so as to polymerize and/or crosslink the main agent. The curing agent is selected depending on the type of the main reactive group in the main agent. An alkenyl group-containing compound is used as the curing agent if the main agent has hydrosilyl groups as its main reactive groups, and a hydrosilyl group-containing compound is used as the curing agent if the main agent has alkenyl groups as its main reactive groups. Among the above elastomers, a styrene-based elastomer and a silicone-based elastomer are preferred, of which a styrene-based elastomer is more preferred.

The elastomer thin film of the insulating sheet 3 may contain other components such as known additives to the extent that the effects of the invention are not impaired. Examples of known additives include an antioxidant, a weathering stabilizer, a heat stabilizer, a lubricant, a crystal nucleating agent, an ultraviolet absorber, a colorant, a surfactant, and a filler. Any one kind of them may be used alone, or two or more kinds of them may be used in combination.

The conductive wiring 4 is formed on one surface of the elongated base material sheet 5. The conductive wiring 4 has, at the location of the attaching part 2, the multi-point electrode parts 4a that will come into contact with the brain surface after attaching the neural electrode 1 to the brain surface. In other words, the multi-point electrode parts 4a are exposed from the hole parts 3a provided on the insulating sheet 3, and secure an electrical connection to the brain surface by being in contact therewith. As for the dimensions, shape, arrangement, etc. of the multi-point electrode parts 4a, the number and dimensions thereof are not particularly limited depending on a target or purpose of use such as those involving human or other animals. The width of each electrode part 4a can be, for example, 20 $\mu$m to 10 mm. The shape of each electrode part 4a is not limited, and can be, for example, round, square, or rectangular. The space between the electrode parts 4a can be, for example, 20 $\mu$m to 20 mm. The electrode parts 4a can be arranged arbitrarily, and may, for example, be arranged in a square or rectangular array. The outer size of the attaching part 2 can be formed in accordance with, for example, the space between the electrode parts 4a.

The conductive wiring 4 is extended in the longitudinal direction of the base material sheet 5 in such a manner that the wiring is continuous from each of the multi-point electrode parts 4a, and that the adjacent wirings are spaced apart from and parallel to each other. The line width and spacing of the conductive wiring 4 are not particularly limited in accordance with, for example, the width and spacing of the electrode parts 4a. Considering the allowable impedance in brain wave measurement, the amount of electric current assumed as that for a subdural electrode, etc., the line width of the conductive wiring 4 can be, for example 20 $\mu$m to 10 mm, and the spacing thereof can be, for example, 20 $\mu$m to 20 mm.

The thickness of the conductive wiring 4 is 10 $\mu$m or smaller. When the thickness of the conductive wiring 4 is 10 $\mu$m or smaller, it is flexible and can be tightly attached to the brain surface. From this perspective, the upper limit of the thickness of the conductive wiring 4 is preferably 5 $\mu$m or smaller, more preferably 1 $\mu$m or smaller. The lower limit of the thickness of the conductive wiring 4 is not particularly limited. From the perspective of electrical conductivity, e.g., impedance, the lower limit of the thickness of the conductive wiring 4 is preferably 200 nm or larger, more preferably 500 nm or larger.

The conductive wiring 4 having the above thickness and line width can be formed on the base material sheet 5 by, for example, a printing method using a conductive ink, or a vapor deposition or sputtering method using conductive nanoparticles. As the conductive ink and conductive nanoparticles, there can be obtained and used those that are commercially available.

Particularly, it is preferred that the conductive wiring 4 is a printed wiring. In the case of a printing method, for example, an inkjet printer is used to perform printing on the base material sheet 5 with the aid of a conductive ink, thereby allowing the conductive wiring 4 to be formed thereon as a wiring pattern. As a method for forming the wiring pattern, other than the inkjet printing described above, the conductive wiring 4 can be formed by being printed on the base material sheet 5, using a simple method such as offset printing and screen printing.

In this specification, "metal nanoparticles" used as a conductive material for forming the conductive wiring 4 refer to metallic particles having a diameter of smaller than 1,000 nm, preferably a diameter of several tens to 300 nm. The term "ink (ink)" is used synonymously with the term "ink (ink)," refers to a dispersion liquid for printing in which a conductive material such as metal nanoparticles is dispersed in a liquid, and may include a conductive paste used for printing conductive materials in the field of printed electronics.

Examples of materials for conductive inks or conductive nanoparticles include, but are not limited to, metals such as gold, platinum, silver, copper, nickel, rhodium, palladium, magnesium, chromium, titanium, and iron, and their oxides; and alloys of these metals and/or their oxides. Any one kind of them may be used alone, or two or more kinds of them may be used in combination. Metal nanoparticles such as gold, platinum, silver, copper, nickel, etc. are especially preferred because these materials are relatively easy to obtain and have low resistivity.

A conductive organic material may, for example, be used as the material of the conductive ink. The conductive organic material can, for example, be selected from conductive carbon materials or conductive polymers. A conductive carbon material can, for example, be selected from conductive carbon black, carbon nanotubes, carbon nanotube derivatives, graphene, graphite, and conductive carbon fibers. Any one kind of them may be used alone, or two or more kinds of them may be used in combination. A conductive polymer may, for example, be selected from polythiophenes, polypyrroles, polyanilines, polyazulenes, polyindoles, polycarbazoles, polyacetylenes, polyfurans, polyparaphenylenevinylenes, polyazulenes, polyparaphenylenes, polyparaphenylene sulfides, polyisothianaphthenes, or polythiazyls. Examples of polythiophenes include PEDOT/PSS (poly(styrenesulfonic acid)) including PEDOT (poly(3,4-ethylenedioxythiophene)).

Although not shown, in an area that is continuously extended from the attaching part 2 toward the opposite side of the multi-point electrode parts 4a, there may be electrically connected a lead wire for drawing a conduction pathway outside the body. Further, there may be provided a connector that is to be connected to the terminals of a measurement device for recording or a stimulator for electrical stimulation.

The base material sheet 5 is an elastomer thin film having a thickness of 100 $\mu$m or smaller. When the thickness of the base material sheet 5 is 100 $\mu$m or smaller, it is flexible and can be tightly attached to the brain surface. From this perspective, the upper limit of the thickness of the base material sheet 5 is preferably 90 $\mu$m or smaller, more preferably 70 $\mu$m or smaller, even more preferably 50 $\mu$m or smaller, and particularly preferably 30 $\mu$m or smaller. The lower limit of the thickness of the base material sheet 5 is not particularly limited. From the perspectives of securing a strength as a base material for the conductive wiring 4, and securing an insulation property to the conductive wiring 4, the lower limit thereof is preferably 3 $\mu$m or larger, more preferably 4 $\mu$m or larger.

An elastomer as the material of the base material sheet 5 is not particularly limited as long as it is safe and compatible for use when in contact with and attached to the brain surface. Specific examples of the elastomer as the material of the base material sheet 5 include those that have been listed above as the examples of the elastomer of the insulating sheet 3; please refer to the corresponding descriptions. Among these examples, a styrene-based elastomer and a silicone-based elastomer can be preferably used. Particularly, a styrene-based elastomer is more preferred due to the fact that if employing a printed wiring as the conductive wiring 4, the base material sheet 5, when hydrophilized, will exhibit a favorable affinity for a conductive ink whereby the conductive wiring 4 can then be formed as a stable wiring pattern in an unbroken manner.

The elastomer thin film of the base material sheet 5 may contain other components such as known additives to the extent that the effects of the invention are not impaired. Examples of such known additives include those that have been listed above as the examples of the additives of the insulating sheet 3.

The base material sheet 5 is preferably hydrophilized on the conductive wiring 4 side. The hydrophilization treatment lowers the contact angle on the surface of the base material sheet 5. In this way, if employing a printed wiring as the conductive wiring 4, a fine conductive wiring can be formed more precisely without having the conductive material-containing ink repelled when performing printing. In other words, by hydrophilizing the base material sheet 5, there will be exhibited a favorable affinity for the conductive ink whereby the conductive wiring 4 can then be formed as a stable wiring pattern in an unbroken manner. Further, there is no need to newly provide an ink receptive layer on the surface of the base material sheet 5.

Although not particularly limited, examples of the hydrophilization treatment may include a treatment using a plasma cleaner, such as an air plasma treatment, an oxygen plasma treatment, a carbon dioxide plasma treatment, a hydrogen plasma treatment, a nitrogen plasma treatment, an argon plasma treatment, and helium plasma treatment; and polyethylene glycol modification, polydopamine modification, and 2-methacryloyloxyethyl phosphorylcholine modification. In an air plasma treatment, it is desired that the treating time is adjusted accordingly to prevent excessive hydrophilization.

The attaching part 2 of the neural electrode 1 preferably has a bending stiffness of $1 \times 10^{-3}$ Nm or lower. When the bending stiffness is small, it is flexible and can be tightly attached to the brain surface. From this perspective, the bending stiffness of the attaching part 2 is preferably $1 \times 10^{-4}$ Nm or lower, more preferably $1 \times 10^{-5}$ Nm or lower, even more preferably $1 \times 10^{-6}$ Nm or lower, and particularly preferably $1 \times 10^{-7}$ Nm or lower. The lower limit of the bending stiffness is not particularly limited. From the perspective of practical use as a neural electrode 1, the lower limit thereof is preferably $1 \times 10^{-10}$ Nm or higher.

A support film 6 supporting the base material sheet 5 is tightly attached to the area that is continuously extended from the attaching part 2 toward the opposite side of the multi-point electrode parts 4a. By employing this support film 6, a stiffness, i.e., moderate rigidity is imparted to the flexible sheet made of the flexible insulating sheet 3 and base material sheet 5, thereby improving handling properties such as those associated with attachment to the brain surface and removal after implantation, and making connection with the lead wire easy.

The material of the support film 6 is not particularly limited as long as it is a resin material that has a moderate flexibility and is safe when attached to the brain surface. A resin material with heat resistance is preferred if connecting the neural electrode 1 to the lead wire. Examples of such resin material include polyimide, polydimethylsiloxane, and a PET film.

The thickness of the support film 6 is preferably 5 to 300 $\mu$m in terms of moderate rigidity and flexibility.

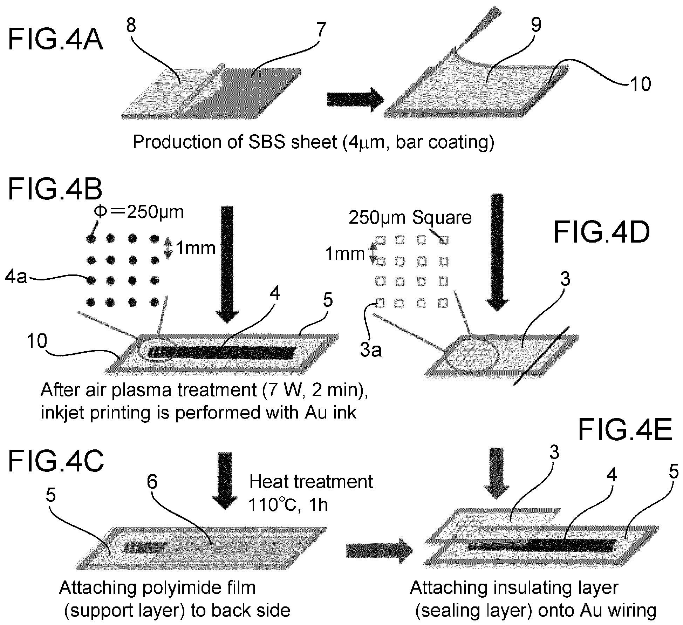

A method for producing the neural electrode 1 of this embodiment is not particularly limited. The neural electrode 1 may, for example, be produced by the following method. FIG.4 is a diagram schematically showing an example of a production procedure of the neural electrode 1.

The insulating sheet 3 and the base material sheet 5, like a SBS thin film 9 in FIG.4(A), can for example be produced by a known film formation method such as a roll-to-roll method using a gravure coater or spin coating. The roll-to-roll technology makes film formation possible at a larger area than when using a spin coater.

For example, an aqueous solution of polyvinyl alcohol (PVA) of a first layer as a sacrificial layer is applied to a polyethylene terephthalate (PET) film 7 serving as a substrate, followed by drying them to form a PVA layer.

As the substrate, there may be used, for example, PET (polyethylene terephthalate), PP (polypropylene), PPE (polyphenylene ether), COP (cycloolefin), PI (polyimide), an aluminum foil, a conductive polymer film, paper, a polysaccharide film, a silicone resin, oblate (gelatin), a silicon wafer, or glass.

The sacrificial layer is used to separate the insulating sheet 3 and base material sheet 5 like the SBS thin film 9 from the substrate after film formation.

Next, a solution that will become an elastomer layer, e.g., a tetrahydrofuran solution of SBS (SBS solution 8) is applied to this sacrificial layer, followed by drying them to allow a second layer to be laminated thereon. In this way, there is produced a laminated film in which the first layer (sacrificial layer) and the second layer (SBS thin film 9) are stacked.

The surface of the SBS thin film 9 serving as the base material sheet 5 is then hydrophilized by air plasma or the like. The conductive wiring 4 having the multi-point electrode parts 4a will then be formed on this hydrophilized surface by printing or the like (FIG.4(B)). The printed wiring formed using a nano-ink is then sintered by heating, if necessary.

A frame such as a tape frame 10 made of paper is then attached to the surface on which the second layer has been formed so as to enclose a necessary shape. Next, by performing peeling from the borders, the two-layered film containing the first and second layers, while being held by the frame, will be peeled away from the substrate (PET film 7). The base material sheet 5 is peeled off together with the conductive wiring 4 using the tape frame 10 made of paper.

By floating the two-layered film held by the paper tape on a pure water with the PVA surface of the first layer (sacrificial layer) being in contact with the pure water, or by immersing such two-layered film in the pure water, only the PVA layer of the first layer will be dissolved, and the nano thin film made of the elastomer layer held by the paper tape will be separated from the substrate.

The support film 6 is to be attached to the back side of the peeled base material sheet 5 (FIG.4(C)).

A laser processing machine or the like is then used to bore the hole parts 3a in the SBS thin film 9 serving as the insulating sheet 3 in such a manner that the sizes of the hole parts 3a will be identical to those of the multi-point electrode parts 4a formed on the base material sheet 5 (FIG.4(D)). The insulating sheet 3 made of the SBS thin film 9 is then laid on top of and tightly attached to the base material sheet 5 so that only the multi-point electrode parts 4a of the conductive wiring 4 are exposed (FIG.4(E)). In order to firmly attach the base material sheet 5 and the insulating sheet 3 to each other, it is preferred that the base material sheet 5 and the insulating sheet 3 are thermo-compression bonded by a method of, for example, fusing the outer circumference of the electrode via a laser processing machine.

The neural electrode 1 of the present invention that has been described above can be used in a method of recording weak electric potentials or electric currents associated with brain activity, or stimulating the brain by transmitting electric currents to the brain, the method including the following steps (A) and (B):

(A) A step of attaching the neural electrode 1 to the brain surface so that the multi-point electrode parts 4a are in contact with the brain surface; and

(B) A step of receiving weak electric potentials or electric currents associated with brain activity by the multi-point electrode parts 4a and recording these electric potentials or electric currents by a measurement device electrically connected to the conductive wiring 4, or stimulating the brain by supplying and transmitting electric currents from a stimulator electrically connected to the conductive wiring 4 to the brain.

In the step (A), for example, the neural electrode 1 may be embedded intracranially. When embedding the neural electrode 1, the cranium of an area where the neural electrode 1 is to be implanted is opened, and the neural electrode 1 is then attached to and implanted on the brain surface so as to establish an electrode arrangement intended. A lead wire may be connected to the neural electrode 1, and this lead wire may then be brought out before closing the cranium and fixed to the scalp or other parts.

In the step (B), for example, the neural electrode 1 is to be connected to the terminals of a measurement device or stimulator via a lead wire or connector, whereby brain wave recording or electrical stimulation can be performed.

According to the neural electrode 1 of the present invention and the above method using the same, based on the extensive and high-density brain wave recording utilizing the advantage of a flexible thin film electrode, although a treatment purpose thereof is not particularly limited, the neural electrode 1 of the present invention may, for example, be applied to an integrated device for diagnosis and treatment of intractable epilepsy.

Clinical practice for epilepsy presents challenges both at the time of diagnosis and at the time of treatment. In diagnosis, the conventional electrodes are thick and the distances between these electrodes are wide, thereby limiting the area of implantation and making sampling errors more likely to occur. In the cases of the current electrodes, they could be dangerous because too many implanted electrodes can cause symptoms of cerebral hypertension, which makes it inevitable to limit the area of implantation.

In addition, current chronic electrode recording is wired, which poses a risk of infection, thereby limiting the recording period and thus resulting in inadequate and incomplete recordings. Due to the limited recording time, patients must reduce the dose of their usual antiepileptic drugs before undergoing chronic intracranial brain wave recording, thereby resulting in brain wave recordings of seizures other than those being treated, which reduces the quality of the diagnosis. The low accuracy in epileptogenic region identification is a contributing factor of the limited treatment outcomes of focal resection.

A challenge at the time of treatment is that the only neuromodulation available in Japan for patients who have difficulty with focal resection is the vagus nerve stimulator (VNS), limiting the range of treatment options. Responsive neurostimulation system (RNS) used in Europe and the U.S. employs dedicated electrodes, and electrodes for recording brain waves have to be replaced for treatment. Further, the number of electrodes that can be used for RNS is limited, and the stimulation effect can only be achieved in a narrow range of brain regions. These are the reasons for the limited treatment outcomes of electrical stimulation therapy.

In the future medical examination flow, as a system providing clinically valuable information according to both diagnostic and treatment purposes, a solution is sought by realizing a remote monitoring system enabled by wireless power supply, remote monitoring, data storage, and automatic data analysis. By using this system, patients can return home during the measurement period, the electrode implantation period is up to 6 months without wired connection, and the brain wave data measurement period can be extended by wireless data transfer. Since the electrode of the present invention is a recording/stimulation electrode that is thinner, lighter, and softer than the conventional electrodes, it enables measurement of brain wave data in a wide range and at a high density where, for example, 128 to 256 channels of electrode are implanted. That is, symptoms of cerebral hypertension will not be observed even by increasing the number of the electrodes implanted, and sampling errors are unlikely to occur because the area of implantation can be widened. The electrode of the present invention is capable of realizing brain wave recording in a wide range and at a high density, stably installing multiple electrodes, and being embedded for a long period of time without compressing brain tissues. Thus, the electrode of the present invention is suitable for embodying a wireless remote monitoring system, which is a goal pursued by the medical examination flow for diagnosis and treatment of intractable epilepsy, etc.

The present invention has been described as above based on its embodiment. However, the invention shall not be limited to this embodiment, but can actually be modified in various ways without departing from the gist of the present invention.

WORKING EXAMPLES

The present invention is described in greater detail hereunder with reference to working examples. However, the invention shall not be limited to these working examples.

<Working example 1> Production of flexible thin film electrode

A bar coater was used to form a thin film (7 wt%, 4 μm) made of a styrene-butadiene-styrene block copolymer (SBS, polystyrene-block-polybutadiene-block-polystyrene by Sigma-Aldrich Japan) on a PET film (Lumirror 25 t60 by PANAC Co., Ltd.) on which a polyvinyl alcohol (7 wt%) (Polyvinyl Alcohol by KANTO CHEMICAL CO., INC.) layer had been formed by gravure printing (FIG.4(A)). The surface of the SBS thin film obtained was treated with a plasma cleaner (PDC-001, HARRICK PLASMA, NY) for hydrophilization (FIG.4(B)). There, a treating time was considered (7W, 0, 1, 2, and 3 min). Under each treating time, a contact angle on the air plasma-treated SBS thin film was measured so as to evaluate the structure of a printed wiring.

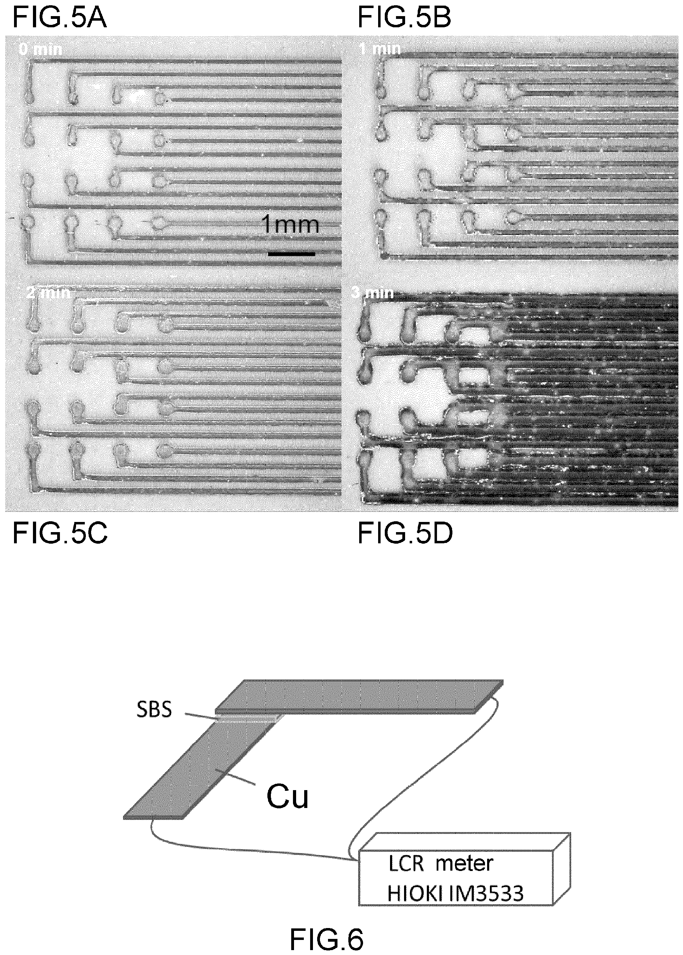

The contact angle on the surface of the SBS thin film became smaller as the time for air plasma treatment increased, indicating that hydrophilization on the surface was facilitated (Table 1). As a result of performing inkjet printing with a gold nano-ink on each plasma-treated thin film, part of the wirings on surfaces untreated with plasma were found to have broken, and wirings blurred on surfaces that had undergone treatment for 3 min (FIG.5). Based on these results, the plasma treatment time for producing the electrode was set to 2 min.

[Table 1]

| Time (min) | Contact angle(°) |
|---|---|
| 0 | 81.0 ± 4 |
| 1 | 60.5 ± 5 |
| 2 | 42.4 ± 3 |
| 3 | 30.8 ± 12 |

Next, using a material printer (Dimatix DMP-2831 by FUJIFILM Corporation), there was printed a multi-point electrode structure having 16 microelectrodes (250 $\mu$m × 250 $\mu$m, 1 mm spacing) made of an Au nano-ink (Au-J by C-INK Co., Ltd.) (FIG.4(B)). After sintering the printed wiring (110°C, 1 hour), the electrodes were peeled off using a paper tape frame. At that time, a polyimide film (Kapton (registered trademark) by DU PONT-TORAY CO., LTD.) was attached to the back side of the peeled sheet as a support (FIG.4(C)).

Next, an SBS thin film of the same thickness as the base material was used as an insulating layer. There, a laser processing machine (VLS2.30DT by Universal Laser Systems, Inc) was used to bore the SBS thin film so as to form a size(s) identical to those of the printed electrodes (250 $\mu$m × 250 $\mu$m, 1 mm spacing) (FIG.4(D)). This insulating layer made of the SBS thin film was then laid on top of the base material so that only measurement portions of the electrodes on the surface of the printed wiring were exposed (FIG.4(E)), followed by using a laser processing machine to fuse the outer circumference of the electrode so as to thermo-compression bond the base material and the insulating film to each other.

<Working example 2> Physical property evaluation

The thicknesses of the SBS thin film and Au wiring of the electrode produced in the working example 1 were measured using a surface profiler (Dektak, Bruker, MA), and a bending stiffness of this thin film electrode was compared to that of an electrode used for comparison. The bending stiffness (D) was defined by the following formula and calculated from a Young's modulus (E), Poisson's ratio ($\gamma$), and film thickness (h) of the electrode material.

[Formula 1]

$$D = \frac{Eh^3}{12(1-\gamma^2)}$$

The bending stiffness is the sum of each component. The electrode produced in the working example 1 exhibited a value of $3.98 \times 10^{-9}$ Nm (Table 2), while the electrode that was used for comparison and made of a silicone (PDMS) thin film and a stainless steel electrode (SUS304) exhibited a value of $2.20 \times 10^{-3}$ Nm. Therefore, it was found that a thin film-shaped electrode had a highly flexible structure in which the bending stiffness was $1.81 \times 10^{-6}$-fold.

[Table 2]

| Electrode of working example | Thickness ($\mu$m) | Young's modulus (Pa) | Poisson's ratio | Bending stiffness (Nm) |
|---|---|---|---|---|
| SBS | 8 | $4.5 \times 10^7$ | 0.5 | $2.56 \times 10^{-9}$ |
| Au | 0.56 | $7.80 \times 10^{10}$ | 0.44 | $1.42 \times 10^{-9}$ |
| Total | | | | **$3.98 \times 10^{-9}$** |

| Electrode for comparison | Thickness ($\mu$m) | Young's modulus (Pa) | Poisson's ratio | Bending stiffness (Nm) |
|---|---|---|---|---|
| Silicone(PDMS) | 100 | $1.32 \times 10^6$ | 0.5 | $1.47 \times 10^{-7}$ |
| Stainless steel(SUS304) | ~50 | $1.93 \times 10^{11}$ | 0.29 | $2.20 \times 10^{-3}$ |
| Total | | | | **$2.20 \times 10^{-3}$** |

<Working example 3> Electric property evaluation

In order to evaluate the insulation performance of the SBS thin films used as the base material and insulating layer of the electrode produced in the working example 1, a resistance value at DC current was measured, and a resistivity was then calculated. A resistivity measurement system shown in FIG.6 was used for measurement. Comparisons were made among SBS thin films having thicknesses of 330 nm, 1 $\mu$m, 1.5 $\mu$m, 2 $\mu$m, 3.7 $\mu$m, and 6.7 $\mu$m.

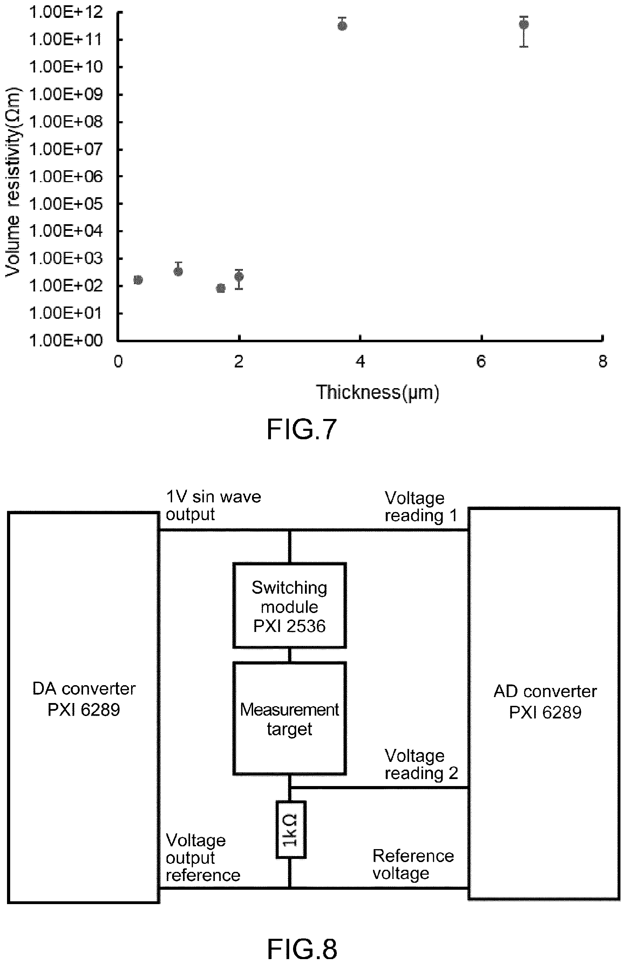

In insulating the printed wiring, the resistance values of SBS thin films with different thicknesses were measured, and it was indicated that a film thickness of 3.7 $\mu$m or larger led to insulating properties (FIG.7). Based on these results, a 3.7 $\mu$m-thick SBS thin film was selected as the electrode protection material having an insulating property while possessing flexibility and conformability to biological tissues.

Next, a resistivity measurement system was established to analyze the resistance of the printed wiring (FIG.8). Specifically, the printed wiring was connected using an FFC conversion connector at a position indicated as "measurement target." The position indicated as "reference voltage" was then used as the ground for analog voltage reading, and analog voltage was read at "voltage reading positions 1 and 2." The voltage output for loading the measurement target was a sine wave with an amplitude of 1 V, where the frequencies were 0.1, 0.2, 0.5, 1, 2, 5, 10, 20, 50, 100, 200, 500, 1k, 2k, 5k, 10k, 20k, and 25 kHz. ADA converter (PXI6289 by National Instruments, TX) was used to read and output analog voltages, and a switching module (PXI2536 by National Instruments, TX) was used to switch targets at the same electrode. The potential difference generated at both ends of the measurement target was determined by calculating the difference between the voltages read, and the current flowing in the circuit was determined from the potential difference between the two ends of a 1 kQ resistor. The resistance was calculated from these potential differences and current. There, the resistivity of the printed wiring was calculated by setting the line width of the printed wiring to 140, 280, 420, 560, 700, 840, and 960 $\mu$m, the number of printed layers to 1, 2, 3, 4, and 5 (280 $\mu$m per layer), and the length of a conducting wire to 5 cm.

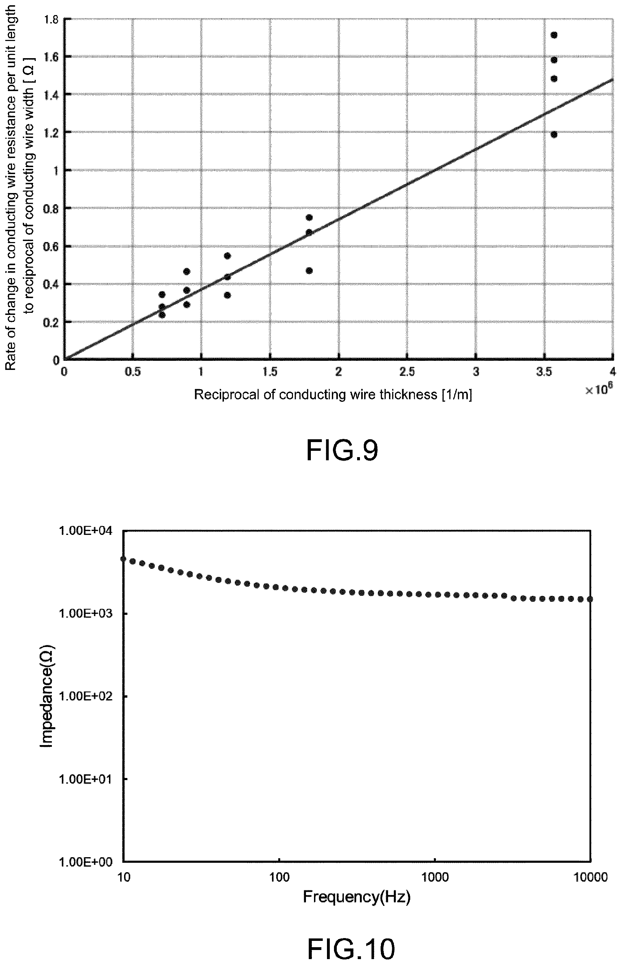

As a result of measuring the resistance of the printed wiring, the volume resistivity was found to be $3.70\times10^{-7}$ Qm (FIG.9). There, the electrode with one printed layer had an unstable structure, which resulted in variations in resistance value. In the cases where two or more layers were subjected to printing, variation was reduced, and a low-resistance wiring was obtained. From these results, there was found a method of forming a conductive wiring whose resistance falls into a desired range by controlling the line width and thickness of a printed wiring.

Finally, the electrochemical impedance of the electrode at 10 Hz to 10 kHz was evaluated using an impedance analyzer (3532-80 by HIOKI E.E. CORPORATION). A phosphate buffer solution (pH 7.6 by FUJIFILM Corporation) was used as a solvent, and an Ag/AgCl electrode was used as a reference electrode.

The electrochemical impedance at 1 kHz of a flexible thin film electrode (electrode part diameter 250 $\mu$m) produced for use on a rat brain surface was 1.68 kQ (FIG. 10). Generally, the impedance in ECoG measurement for human is desirably <20 kQ ("Revised Standards for Clinical Electroencephalography" (2002) by Japanese Society of Clinical Neurophysiology), and the electrode of the present invention is expected to be within an optimal measurement range.

<Working example 4> In vivo recording of nerve potentials

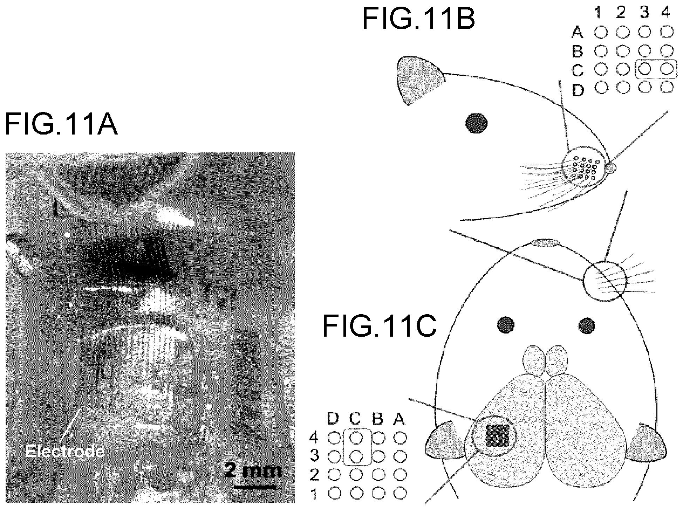

The thin film electrode was attached to the brain surface of an anesthetized rat, and there was evaluated a correlation between a mechanical stimulation to each whisker and ECoG, utilizing a whisker-brain response circuit (FIG. 11).

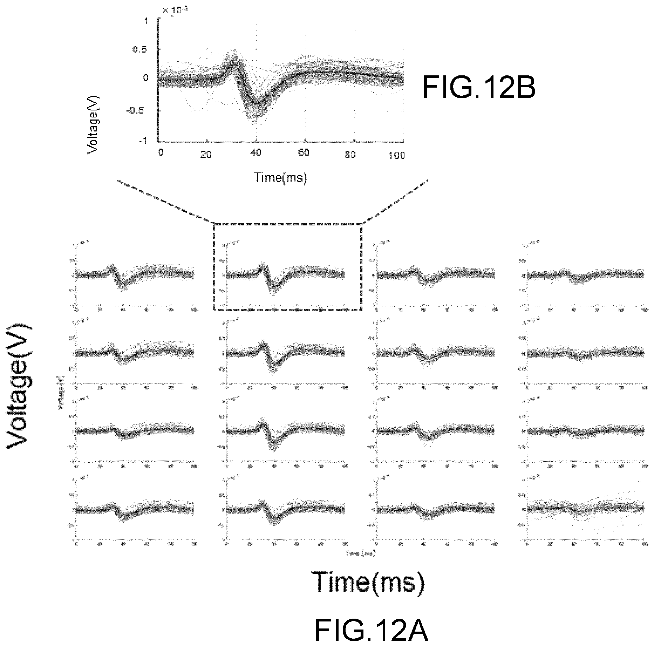

With the thin film electrode having an excellent conformability to the brain surface, ECoG was able to be successfully measured in a stable manner (FIG. 12). There, as a result of examining the spatial resolution of the electrode based on the whisker-brain response circuit, between any whisker stimulation site and the multi-point electrode array (4$\times$4), there was found a column-wise correspondence. This result in in vivo environment indicates that the recording of nerve potentials using the thin film electrode of the working example can also be applied to humans.

<Working example 5> In vitro electrical stimulation

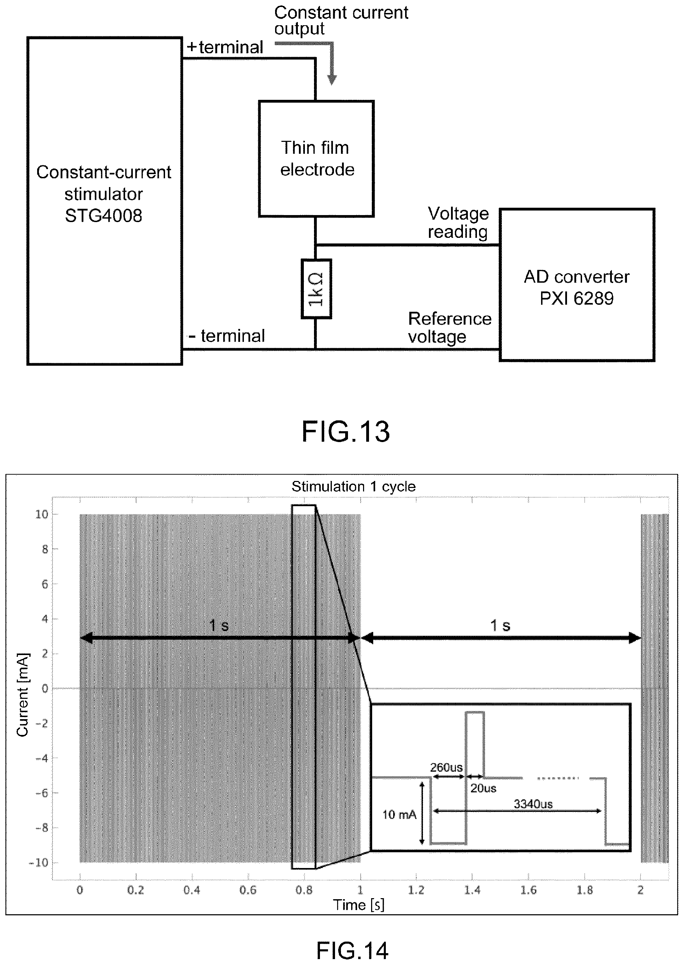

A current load test system was established in which the measurement target was the current output from a constant-current stimulator (FIG. 13). Specifically, the constant current output from the + terminal flows to the - terminal through a 1 kQ resistor. There, the current output was confirmed by measuring the potential difference between the two ends of the 1k$\Omega$ resistor using an AD converter (PXI6289 by National Instruments, TX). Here, when measuring the potential difference, the output waveform was programmed using a constant-current stimulator (STG4008-16 mA by Multi Channel Systems, Baden-Wuerttemberg (Germany)). Each cycle was composed of 1 sec of stimulation and 1 sec of rest, and this was repeated for 900 cycles. For a single output waveform, the amplitude was set to 10 mA, and the frequency was set to about 300 Hz. In the current load test, that a 10 mA current was flowing at a one-layered electrode having a 140 $\mu$m-wide conducting wire was confirmed by an AD converter (FIG. 14).

Further, the electrode was attached to the motor area of the rat brain surface under anesthesia, and the behavior of the rat was observed when electrically stimulated by the constant-current stimulator (STG4008 by Multi Channel Systems, Baden-Wuerttemberg (Germany)). The rat was found to be able to move their arms and whiskers in a site-specific manner as a result of attaching the electrode to the motor area of its brain surface and then applying an electric current thereto.

The area of one whisker in the sensory area of the left brain of the anesthetized rat was subjected to current stimulation using this electrode, and the movement induced by the electrical stimulation was evaluated by recording the electromyogram (EMG) at the root on the left side of the rat being stimulated. Here, a square wave current of 1.6 mA was intermittently applied five times (duration of square wave: 260 $\mu$s, time interval between square waves: 3,340 $\mu$s) for stimulation.

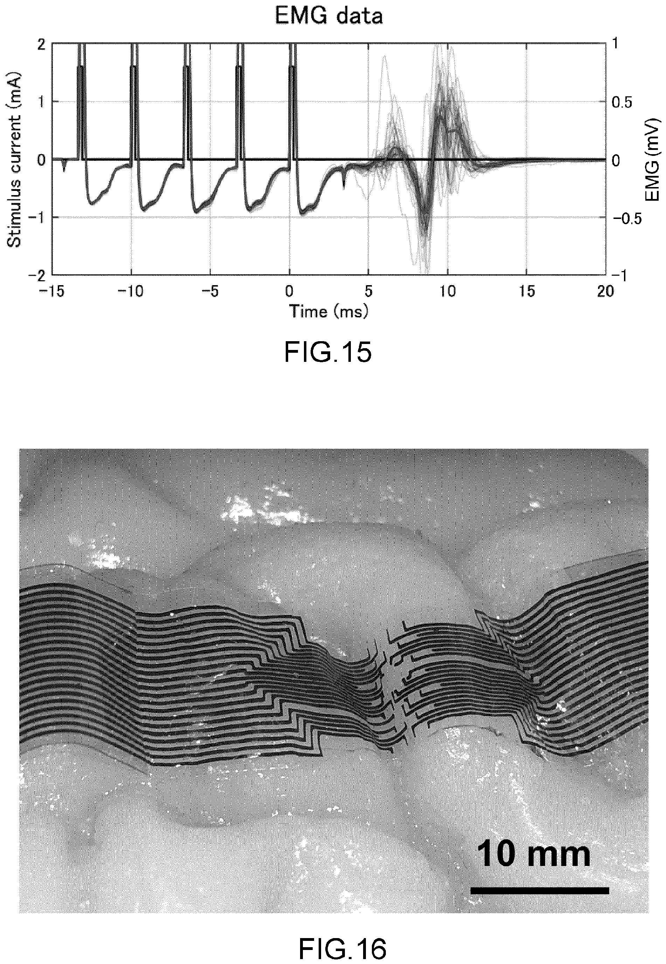

That the whiskers were being electrically stimulated was confirmed by recording the EMG at the root of one whisker (FIG. 15). EMG was recorded between 5-15 ms, following artifacts related to the stimulation current and artifacts related to the on/off of the isolator output (potential changes recorded at -14.4 ms and 3.34 ms).

These results indicate that electrical stimulation to the brain surface using the thin film electrodes of the working example can be applied to humans.

As described above, the electrode produced was able to measure nerve potentials by being attached to the brain surface of a rat. Further, waveforms when stimulating the whiskers were also able to be recorded. Further, it was verified that a similar thin film electrode was able to be attached to the brain surface for electrical stimulation.

<Working example 6> Attachment to brain gel model of human size

The electrode of the present invention was attached to a brain gel model of human size (MR. Brain by ASTEC CO.,LTD. (headquartered in Higashimatsuyama City, Saitama Prefecture)) (FIG. 16). This brain gel model is a product intended for anastomosis practice or the like by physicians, and is soft and elastic with an uneven surface that imitates the sulcus of the brain. It was confirmed that the electrode of the present invention conformed with the surface irregularities that imitated the brain sulcus. That is, it was indicated that since the electrode of the present invention is thin, light, and soft, it can be embedded for a long period of time without compressing brain tissues and used to measure brain wave data in a wide range and at a high density even when employing a wider area of implantation and a larger number of electrodes implanted.

DESCRIPTION OF THE SYMBOLS

    1: Neural electrode
    2: Attaching part
    3: Insulating sheet
    3a: Hole part of insulating sheet
    4: Conductive wiring
    4a: Multi-point electrode part
    5: Base material sheet
    6: Support film
    7: PET film
    8: SBS solution
    9: SBS thin film
    10: Paper tape frame

**Claims**

1.  A recording and/or stimulation neural electrode with an attaching part thereof as a sheet-shaped electrode being attached to the brain surface upon use, wherein

    the attaching part has at least:

    an insulating sheet whose one surface serves as an attaching surface to be attached to the brain surface; and a conductive wiring formed on a surface of the insulating sheet that is opposite to the attaching surface, wherein

    multi-point electrode parts of the conductive wiring are exposed from hole parts of the insulating sheet, and wherein
    the insulating sheet is an elastomer thin film having a thickness of 2 to 100 $\mu$m, and the conductive wiring has a thickness of 10 $\mu$m or smaller.

2.  The recording and/or stimulation neural electrode according to claim 1, wherein the neural electrode has a base material sheet on which the conductive wiring is formed, the base material sheet is tightly attached to the insulating sheet across the conductive wiring at the attaching part, and the base material sheet is an elastomer thin film having a thickness of 100 $\mu$m or smaller.

3. The recording and/or stimulation neural electrode according to claim 2, wherein the conductive wiring is a printed wiring.

4. The recording and/or stimulation neural electrode according to claim 3, wherein the base material sheet is hydrophilized on the conductive wiring side.

5. The recording and/or stimulation neural electrode according to any one of claims 1 to 4, wherein the insulating sheet is a styrene-based elastomer.

6. The recording and/or stimulation neural electrode according to any one of claims 1 to 5, wherein the attaching part has a bending stiffness of $1 \times 10^{-3}$ Nm or lower.

7. The recording and/or stimulation neural electrode according to any one of claims 1 to 6, wherein a support film supporting the base material sheet is tightly attached to an area that is continuously extended from the attaching part toward the opposite side of the multi-point electrode parts.

8. A method of recording weak electric potentials or electric currents associated with brain activity, or stimulating the brain by transmitting electric currents to the brain, comprising:

a step of attaching the neural electrode according to any one of claims 1 to 7 to the brain surface so that the multi-point electrode parts are in contact with the brain surface; and
a step of receiving weak electric potentials or electric currents associated with brain activity by the multi-point electrode parts and recording the electric potentials or electric currents by a measurement device electrically connected to the conductive wiring, or stimulating the brain by supplying and transmitting electric currents from a stimulator electrically connected to the conductive wiring to the brain.

FIG.1

4

1

2

3

A A

5

B B

FIG.2

4

1

3a

4a

2

3

FIG.3A

FIG.3B

FIG.4A

Production of SBS sheet (4μm, bar coating)

FIG.4B

Φ=250μm

1mm

After air plasma treatment (7 W, 2 min),
inkjet printing is performed with Au ink

250μm Square

1mm

FIG.4D

FIG.4C

Heat treatment
110°C, 1h

Attaching polyimide film
(support layer) to back side

FIG.4E

Attaching insulating layer
(sealing layer) onto Au wiring

FIG.5A

FIG.5B

FIG.5C

FIG.5D

1mm

FIG.6

FIG.7

FIG.8

FIG.9

FIG.10

FIG.11A

FIG.11B

FIG.11C

FIG.12B

Voltage(V)

Time(ms)

FIG.12A

FIG.13

FIG.14

## EMG data

FIG.15

FIG.16

## INTERNATIONAL SEARCH REPORT

| International application No. |
|---|
| **PCT/JP2022/035817** |

**A. CLASSIFICATION OF SUBJECT MATTER**

*A61B 5/37*(2021.01)i; *A61B 5/271*(2021.01)i; *A61B 5/293*(2021.01)i; *A61N 1/05*(2006.01)i; *A61N 1/36*(2006.01)i
FI: A61B5/37; A61B5/271; A61B5/293; A61N1/05; A61N1/36

According to International Patent Classification (IPC) or to both national classification and IPC

**B. FIELDS SEARCHED**

Minimum documentation searched (classification system followed by classification symbols)

A61B5/37; A61B5/271; A61B5/293; A61N1/05; A61N1/36

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2009-45368 A (OSAKA UNIV) 05 March 2009 (2009-03-05)<br>paragraphs [0032], [0034], [0036]-[0037], [0055], [0061], [0078], [0089] | 1-8 |
| Y | JP 2012-228375 A (KYOTO SANGYO UNIV) 22 November 2012 (2012-11-22)<br>paragraphs [0003], [0025], [0029] | 1-8 |
| Y | JP 2015-221177 A (UNIV YAMAGUCHI) 10 December 2015 (2015-12-10)<br>paragraph [0020] | 1-8 |
| A | 山岸健人、藤枝俊宣, 高分子ナノ薄膜を用いた皮膚貼付型ウェアラブルデバイス「セカンドスキン・エレクトロニクス」の開発, エレクトロニクス実装学会誌, August 2021, vol. 24, no. 5, 353-360<br>p. 353, fig. 1, p. 359, "4. Conclusion and future development", lines 1-3, 17,<br>(YAMAGUISHI, Kento. FUJIE, Toshinori. Polymeric Nanofilm-Based Skin-Interfaced Wearable Devices "Second-Skin Electronics". Journal of The Japan Institute of Electronics Packaging.) | 1-8 |

☐ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | |
| "O" | document referring to an oral disclosure, use, exhibition or other means | "Y" | document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" | document member of the same patent family |

| Date of the actual completion of the international search | Date of mailing of the international search report |
|---|---|
| **12 October 2022** | **27 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/035817**

| Patent document cited in search report | | | Publication date (day/month/year) | Patent family member(s) | Publication date (day/month/year) |
|---|---|---|---|---|---|
| JP | 2009-45368 | A | 05 March 2009 | US 2009/0228066 A1 paragraphs [0081] [0083], [0086]-[0087], [0106], [0136], [0155], [0166] | |
| JP | 2012-228375 | A | 22 November 2012 | (Family: none) | |
| JP | 2015-221177 | A | 10 December 2015 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016181958 A

**Non-patent literature cited in the description**

- *NATURE MATERIALS,* June 2010, vol. 9, www.nature.com/naturematerials
- *Scientific Reports,* 2018, vol. 8, 3825
- *Scientific Reports,* 2019, vol. 9, 13379, https://doi.org/10.1038/s41598-019-49772-z
- **2002.** Revised Standards for Clinical Electroencephalography. Japanese Society of Clinical Neurophysiology